# EUROPÄISCHE PATENTSCHRIFT

(11) **EP 2 231 053 B1**
(45) Veröffentlichungstag und Bekanntmachung des Hinweises auf die Patenterteilung: **22.07.2015**
(21) Anmeldenummer: 09704141.2
(22) Anmeldetag: 20.01.2009
(51) Int. Cl.: A61B 19/08, B25J 19/00

(54) **STERILBARRIERE FÜR EINEN CHIRURGISCHEN ROBOTER MIT DREHMOMENTSENSOREN**
STERILE BARRIER FOR A SURGICAL ROBOT WITH TORQUE SENSORS
BARRIÈRE STÉRILE POUR ROBOT CHIRURGICAL AVEC CAPTEURS DE COUPLE DE ROTATION

(30) Priorität: 24.01.2008 DE 102008005901
(43) Veröffentlichungstag der Anmeldung: 29.09.2010
(73) Patentinhaber: KUKA Laboratories GmbH, 86165 Augsburg (DE)
(72) Erfinder: ORTMAIER, Tobias, 30 966 Hemmingen (DE); JACOB, Dirk, 86399 Bobingen (DE); NEFF, Thomas, 74080 Heilbronn (DE); HEINZE, Achim, 86720 Nördlingen (DE)
(74) Vertreter: Patentanwälte Funk & Böss GbR
(86) Internationale Anmeldenummer: PCT/EP2009/050577
(87) Internationale Veröffentlichungsnummer: WO 2009/092701

(56) Entgegenhaltungen:
- EP-A- 1 754 448
- WO-A-99/08841
- WO-A-03/037755
- WO-A-2007/122717
- DE-U1-202007 006 121
- FR-A- 2 683 479
- US-A1- 2002 111 713

## Beschreibung

Die Erfindung betrifft eine Sterilbarriere nach Anspruch 1.

Chirurgische Roboter sind Handhabungsmaschinen, die zur selbsttätigen und/oder gesteuerten Ausführung von chirurgischen Arbeitsvorgängen an Patienten in der Human- und/oder Veterinärmedizin mit zweckdienlichen Werkzeugen, zum Beispiel an einem so genannten Handflansch, ausgerüstet und in mehreren Bewegungsachsen insbesondere hinsichtlich Orientierung, Position und Arbeitsablauf programmierbar bzw. steuerbar sind.

Bei Operationen wird häufig zusätzlich zum normalen Operationsbesteck immer häufiger technisches Gerät, zum Beispiel Endoskop, OP-Mikroskop, Bohrmaschine, eingesetzt. Der Einsatz unterliegt strengen Hygieneanforderungen. Sobald im Verlauf einer Operation ein derartiges Gerät zum Einsatz kommt und - wenn auch nur indirekt - Kontakt zum Patienten hergestellt wird, muss die Sterilität der Kontaktoberfläche gewährleistet werden. Die Kontaktoberfläche ist im Normalfall die gesamte äußere Oberfläche des Geräts. Derzeitige Sterilisierungstechniken (z.B. Dampfsterilisierung) sind für elektrische und elektronische Geräte nicht bzw. nur bedingt geeignet. Um trotzdem die Sterilität zu erreichen, werden im Allgemeinen sterile Plastik- oder Gummihüllen, so genannte Sterilbarrieren, über das (an sich nicht sterile) Gerät gezogen, bzw. das Gerät wird mit solchen steril abgedeckt.

Bei chirurgischen Robotern tritt dabei das Problem auf, dass der Roboter durch die sterile Hülle in seiner Bewegungsfreiheit nicht eingeschränkt werden darf. Damit die Gefahr des Reißens der Hülle ausgeschlossen wird, kann die Hülle zum Beispiel sehr weit ausgestaltet sein. Dies hat zur Folge, dass die Hülle in den Arbeitsbereich des Chirurgen hineinragen kann. Deshalb wird sie dabei häufig mit Klebebändern zusätzlich möglichst nah am Roboter fixiert.

Wenn ein chirurgischer Roboter mit Drehmomentsensoren (DMS) in den Gelenken ausgestattet ist, können durch zu enge Hüllen oder mit Klebebändern veränderte Hüllen zusätzliche a priori nicht bekannte Momente auf die Drehmomentsensoren einwirken, dies gilt insbesondere bei Bewegungen des Roboters. Daraus resultiert, dass zum Beispiel eine Gravitationskompensation, welche sowohl die Messwerte der Gelenkwin-kelsensoren als auch der Drehmomentsensoren verwendet, fehlerhafte Ergebnisse liefern kann. Auch Regelungsverfahren, wie zum Beispiel Impedanz-, Kraft- oder Admittanzregelung, können durch fehlerbehaftete DMS-Werte nicht mehr oder nur sehr eingeschränkt funktionieren. Durch eine Sterilbarriere für solch einen Roboter dürfen deshalb im Verlauf einer Operation bei allen möglichen relevanten Lagen/Konfigurationen des Roboters keine zusätzlichen, insbesondere keine unberechenbaren oder abschätzbaren, oder nur sehr geringe Momente auf die Drehmomentsensoren einwirken.

Beispiele für Sterilbarrieren bei chirurgischen Robotern beschreiben zum Beispiel die Dokumente US 20060161138 A1 und US 020060235436 A1. Auf die Problematik der Drehmomentsensoren gehen diese Schriften nicht ein.

Eine Sterilbarriere nach dem Oberbegriff des Anspruchs 1 wird in WO 99/08841 offenbart.

Die Aufgabe der Erfindung ist es daher, eine Sterilbarriere für chirurgische Roboter mit Drehmomentsensoren anzugeben, welche im Vergleich zum Stand der Technik die obigen Nachteile behebt bzw. bedeutend reduziert.

Eine weitere Aufgabe der Erfindung ist es, einen entsprechenden chirurgischen Roboter anzugeben.

Die Aufgabe der Erfindung wird gelöst durch eine Sterilbarriere für einen chirurgischen Roboter mit zumindest einem Gelenk mit zwei gegenüber liegenden, um eine gemeinsame Gelenkachse relativ zueinander drehbaren Gelenkkörpern und einem Drehmomentsensor, aufweisend:
zumindest zwei Sterilbarrierenabschnitte mit jeweils einem Endabschnitt zur dichten Anbringung an jeweils einem Gelenkendabschnitt der Gelenkkörper; und
eine Dichtungsanordnung zur Bildung einer durchgehenden sterilen und dichten Drehverbindung der Endabschnitte der zumindest zwei Sterilbarrierenabschnitte.

Hierdurch wird die uneingeschränkte Beweglichkeit des chirurgischen Roboters bei gleichzeitig ausgeschlossener bzw. nahezu ausgeschlossener Rückwirkung auf die Drehmomentsensoren in jeder relevanten Roboterlage/-konfiguration gewährleistet. Des Weiteren wird ein optimiertes, das heißt zeitsparendes, Anlegen und Abziehen der Sterilbarriere erzielt.

Durch den Einsatz einer zumindest zweiteiligen Sterilbarriere aus Kunststoff oder Gummi (so genannte Drapes), deren Einzelabschnitte jeweils vor und nach den Positionen der Drehmomentsensoren auf der Gehäuseoberfläche ansetzen, kann ein Einfluss der Hülle auf diese Sensoren verhindert bzw. reduziert werden. Damit die gesamte Roboteroberfläche mit der Sterilbarriere überzogen werden kann, müssen die Lücken im Bereich der Gelenke mit den Drehmomentsensoren zwischen den oben genannten Einzelabschnitten der Sterilbarriere überbrückt werden.

Dazu weist die Dichtungsanordnung Folgendes auf:
einen Innenring zur dichten Anbringung auf dem Gelenkendabschnitt des ersten Gelenkkörpers des Gelenks;
einen Außenring mit einem Anbringungsabschnitt zur Anbringung auf dem Gelenkendabschnitt des zweiten Gelenkkörpers des Gelenks und mit einem zur Überlappung des Innenrings vorgesehenen Dichtabschnitt; und
ein zur Anordnung zwischen dem Innenring und dem Dichtabschnitt des Außenrings vorgesehenes Dichtelement zur Abdichtung des Innenrings gegenüber dem Außenring.

Der Außenring reicht über den Gelenkspalt und über den Innenring hinüber und hat einen Abschnitt mit einem größeren Durchmesser als der Innenring. Dadurch entsteht zwischen Innen- und Außenring eine Überlappung, welche durch ein Dichtelement, zum Beispiel ein steriler Dichtring aus Gummi, verschlossen wird. Die Sterilbarriere des Roboters ist damit durchgängig.

Für eine schnelle Montage und Demontage weisen der Innenring und der Außenring jeweils eine Verschlusseinrichtung zum Befestigen und Lösen auf.

Die beiden Ringe sind beim Einsatz des Roboters gegeneinander drehbeweglich, wobei durch die Reibung des Dichtelementes die Messwerte der Drehmomentsensoren nicht verfälscht werden. Dazu können zum Beispiel zur Verringerung der Reibung und gleichzeitig zur Erhöhung der Dichtigkeit die Innenseite des Dichtabschnitts des Außenrings und die Außenseite des Innenrings, im Bereich der Auflagefläche für das Dichtelement mit einer geeigneten Beschichtung und/oder O-berflächenbearbeitung versehen sein. Durch Verwendung geeigneter Störgrößenbeobachter in der Regelung kann die zusätzliche Reibung, welche bei Bewegung der Ringe mit dem Dichtelement untereinander entsteht, identifiziert und in der Regelung kompensiert/berücksichtigt werden.

Um die notwendige Flexibilität der Sterilbarrierenabschnitte zur erhalten, können sie im Bereich von Gelenken oder bewegten Teilen weiter gehalten werden. Dadurch wird ein Spannen oder Reißen beim maximalen Knickwinkel verhindert. Im Bereich von Verbindungselementen, in welchem ein Benutzer den Roboter zur manuellen Führungen bzw. Steuerung anfasst, zum Beispiel um die Konfiguration zu ändern, können die Sterilbarrieren enger gehalten werden.

Die Anfangs- und Endpositionen der Sterilbarrierenabschnitte müssen exakt definiert sein, um eine durchgehende sterile Hülle zu garantieren. Dazu können die Endabschnitte der Sterilbarrierenabschnitte jeweils mit zumindest einem flexiblen Spannelement zur Fixierung und Zusammenwirkung mit einer korrespondierenden Ausnehmung an dem jeweiligen Gelenkendabschnitt ausgebildet sein. Diese Ausnehmungen sind zum Beispiel umlaufende Rinnen oder Nuten, in denen die, zum Beispiel mit Gummizügen ausgestatteten, Endabschnitte aufgenommen werden.

Das gleiche Problem ergibt sich bei der exakten Positionierung der Dichtungsanordnung. Hierzu ist es bevorzugt, dass der Innenring und der Außenring jeweils eine umlaufende Nase zur Zusammenwirkung mit einer jeweiligen Nut an dem korrespondierenden Gelenkendabschnitt aufweisen. Die Nase kann zum Beispiel trapezförmig im Querschnitt ausgebildet sein. Dadurch wird ein schnelles exaktes Positionieren der Dichtungsanordnung durch selbständiges Einziehen der Ringe in die Nuten erreicht, sowie eine Abdichtung erhöht.

Die Endabschnitte der Sterilbarrierenabschnitte können auch zur Zusammenwirkung mit korrespondierenden Anbringungsabschnitten des Innenrings und Außenrings zur Abdichtung und Befestigung an letzteren und den jeweiligen Gelenkendabschnitten vorgesehen sein, indem die Endabschnitte zusammen mit den Nasen in die trapezförmigen Nuten eingebracht werden und von den Nasen in die Nuten gedrückt werden, wobei eine Abdichtung erzielt wird.

Der Innenring und Außenring sind vorzugsweise aus einem sterilisierbaren Werkstoff und/oder einer Kombination aus sterilisierbaren Werkstoffen ausgebildet, zum Beispiel aus einem geeigneten Metall wie Edelstahl. Sie können auch mit Federelementen oder federnden Abschnitten versehen sein, um eine Spannkraft zur Abdichtung und Festlegung der Sterilbarrierenendabschnitte zu erhöhen. In einer weiteren bevorzugten Ausführungsform werden diese Elemente als Einweg- bzw. Verbrauchsartikel hergestellt.

Das Dichtelement kann alternativ mit dem Innenring oder mit dem Außenring fest verbunden sein. Auch ein steriler Zuschnitt oder ein sterilisierbarer Zuschnitt einer Endlosware ist möglich.

Ein erfindungsgemäßer chirurgischer Roboter mit zumindest einem Gelenk mit einem Drehmomentsensor weist zumindest eine oben beschriebene Sterilbarriere auf.

Ausführungsbeispiele der Erfindung sind exemplarisch in den beigefügten schematischen Zeichnungen dargestellt. Es zeigen:
- Fig. 1: eine beispielhafte schematische Darstellung eines chirurgischen Roboters mit einer erfindungsgemäßen Sterilbarriere;
- Fig. 2: eine vergrößerte Teilschnittansicht des Bereichs A eines Gelenks des chirurgischen Roboters nach Fig. 1; und
- Fig. 3: eine vergrößerte schematische Teilschnittansicht des Bereichs B des Gelenks nach Fig. 2.

Gleiche Bauelemente bzw. Bauelemente mit gleicher Funktion sind in den Figuren mit gleichen Bezugszeichen angegeben.

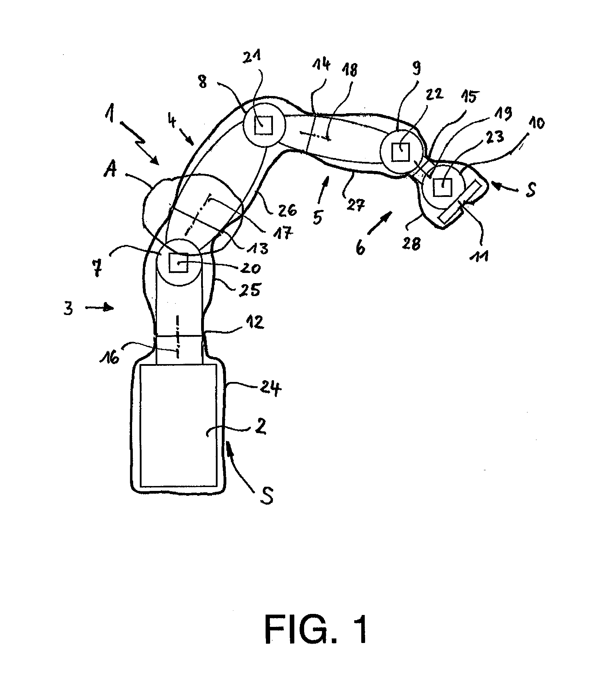

In Fig. 1 ist ein Ausführungsbeispiel eines chirurgischen Roboters (1) mit einer erfindungsgemäßen Sterilbarriere S dargestellt.

Der Roboter 1 weist in diesem Beispiel 8 Achsen auf: vier Gelenkachsen 16 bis 19 und vier Knickgelenkachsen 20 bis 23.

Achse 16 ist eine vertikale Drehachse eines Sockels 2 in Bezug auf eine Basis 3 des Roboters 1. Knickgelenkachse 20 verbindet in einem ersten Knickgelenk 7 quer zur Achse 16 die Basis 3 mit einem ersten Arm 4. Der Arm 4 ist in zwei Teile unterteilt, welche in ihrer Längsachse um die Achse 17 drehbar sind. Das andere Ende des ersten Arms 4 ist mit einem zweiten Knickgelenk 8 schwenkbar um die zweite Knickgelenkachse 21 mit einem zweiten Arm 5 verbunden, welcher wie der erste Arm 4 aus zwei Teilen besteht, die um die Achse 18 in ihrer Längsachse drehbar sind. An dem anderen Ende des zweiten Arms 5 ist in ebensolcher Weise ein drittes Knickgelenk 9 mit einer Knickgelenkachse 22 angebracht, welches über ein zweiteiliges Handstück 6 mit einem Handgelenk 10 mit Knickgelenkachse 23 verbunden ist. Auch das Handstück 6 weist hier zwei Teile auf, die in ihrer Längsachse um die vierte Achse 19, das heißt eine Handgelenkachse, drehbar verbunden sind. An dem Handgelenk 10 ist ein Handflansch 11 für ein nicht gezeigtes Werkzeug angeordnet.

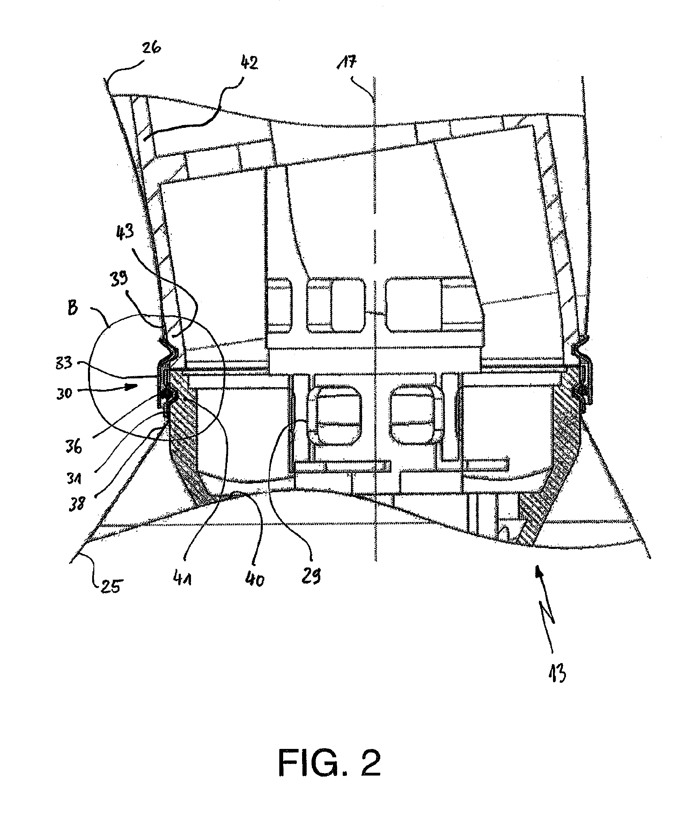

Für den Einsatz in einem OP-Raum ist der Roboter 1 mit einer sterilen Hülle, einer erfindungsgemäßen Sterilbarriere S, vollständig überzogen. Die Sterilbarriere S übt keine unerwünschten Rückwirkungen auf Drehmomentsensoren 29 aus, die in den Gelenkachsen 16 bis 19 jeweils angeordnet sind, wie in Fig. 2 zu sehen ist. Hierzu ist die Sterilbarriere S mehrteilig, in diesem Beispiel mit fünf, Sterilbarrierenabschnitten 24 bis 28 ausgeführt, die jeweils einen Roboterabschnitt umhüllen und in den Gelenken 12, 13, 14, 15 jeweils an den Enden der Gelenke 12 bis 15 angebracht sind. Ein erster Sterilbarrierenabschnitt 24 umhüllt den Sockel 2 und das Unterteil der Basis 3, ein zweiter Sterilbarrierenabschnitt 25 umhüllt das Oberteil der Basis 3, das erste Knickgelenk 7 und das Unterteil des ersten Arms 4, ein dritter Sterilbarrierenabschnitt 26 umhüllt den das Oberteil des ersten Arms 4 und das Unterteil des zweiten Arms 5 mit dem zweiten Knickgelenk 8, ein vierter Sterilbarrierenabschnitt 27 umhüllt das Oberteil des zweiten Arms 5 mit dem dritten Knickgelenk 9 und das Unterteil des Handstücks 10, und ein fünfter Sterilbarrierenabschnitt 28 umhüllt schließlich das Oberteil des Handstücks 10 und das Handgelenk 19 mit dem Handflansch 11. Somit können sich die einzelnen Roboterabschnitte, zum Beispiel das Oberteil des Arms 4 mit dem zweiten Knickgelenk 8 und den daran angebrachten weiteren Abschnitten gegenüber dem Unterteil des Arms 4 verdrehen, ohne dass die Sterilbarrierenabschnitte, hier 25 und 26, verwunden werden und die Drehmomentsensoren 29 beeinflussen. Dies wird nun eingehender im Zusammenhang mit Fig. 2 beschrieben.

Fig. 2 zeigt eine vergrößerte Teilschnittansicht des Bereichs A als beispielhafte Darstellung des Gelenks 13 des chirurgischen Roboters 1 nach Fig. 1.

In dem Gelenk 13 sind das Unterteil des ersten Arms 4 (siehe Fig. 1) als ein erster Gelenkkörper 40 mit einem Gelenkendabschnitt 41 und das Oberteil des ersten Arms 4 als ein zweiter Gelenkkörper 42 mit einem Gelenkendabschnitt 43 um die Gelenkachse 17 drehbar verbunden. Auf der Gelenkachse 17 zwischen den beiden Gelenkendabschnitten 41 und 43, welche sich gegenüber stehen, ist der nicht näher beschriebene Drehmomentsensor 29 angeordnet.

An den Gelenkendabschnitten 41 und 43 sind Endabschnitte 38 und 39 der zweiten und dritten Sterilbarrierenabschnitte 25 und 26 im Bereich einer Dichtungsanordnung 30 dicht angebracht. Die Dichtungsanordnung 30 weist einen Innenring 31 auf, der hier auf dem Gelenkendabschnitt 41 in einer Nut aufgebracht ist. Auf dem gegenüber liegenden Gelenkendabschnitt 43 ist in ähnlicher Weise ein Außenring 33 der Dichtungsanordnung 30 in einer weiteren Nut befestigt. Der Außenring 33 überlappt den Innenring 31 in Richtung der Gelenkachse 17 um eine bestimmte Länge und weist in diesem Bereich einen Durchmesser auf, der größer ist als der Außendurchmesser des Innenrings 31. In diesem Bereich ist ein Dichtelement 36 angeordnet.

Die Endabschnitte 38 und 39 der Sterilbarrierenabschnitte 25 und 26 sind jeweils unter dem Innenring 31 und Außenring 33 der Dichtungsanordnung 30 auf den Gelenkendabschnitten 41 und 43 angebracht, so dass eine dichte und sterile Überbrückung des Gelenks 13 gebildet ist. Die Gelenkkörper 40 und 42 sind gegeneinander verdrehbar, wobei sich die auf ihnen angeordneten Sterilbarrieren 25 und 26 und Ringe der Dichtungsanordnung 30 mitdrehen. Zwischen den Ringen und dem Dichtelement 36 herrscht ein berechenbarer bzw. identifizierbarer Reibungswert, der mit der Drehgeschwindigkeit in einen Regelungsvorgang mit einbezogen werden kann, wodurch die durch die Reibungskräfte hervorgerufenen Störungen der Messwerte des Drehmomentsensors 29 im Wesentlichen kompensierbar sind.

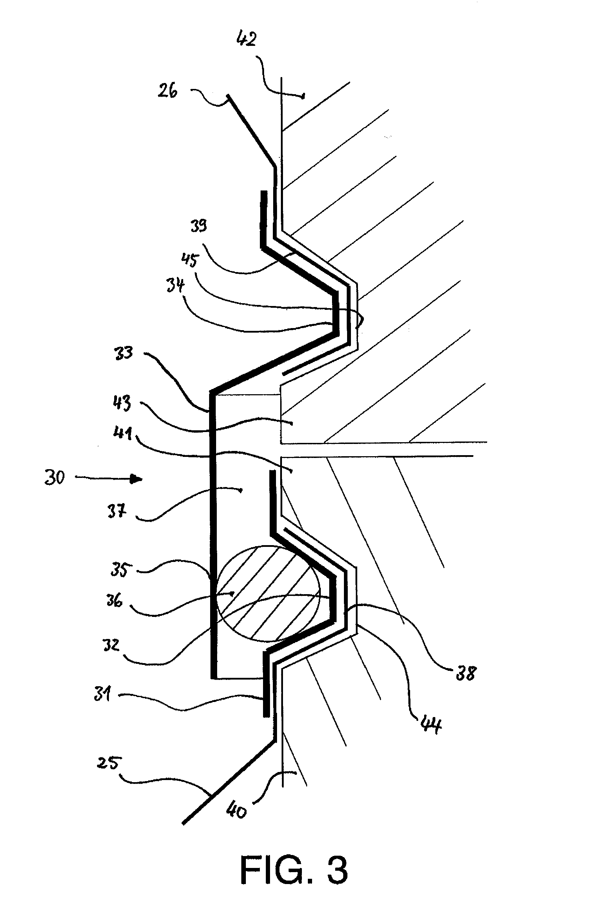

Die Dichtungsanordnung 30 in dem Bereich B ist in Fig. 3 vergrößert gezeigt.

In Fig. 3 ist ein Ausführungsbeispiel dargestellt, in welchem deutlich zu sehen ist, dass die Endabschnitte 38 und 39 der Sterilbarrierenabschnitte 25 und 26 jeweils in einer trapezförmigen Nut 44, 45 unter trapezförmigen Nasen der Ringe 31 und 33 dicht befestigt sind. In einem Anbringungsabschnitt 32 der Nase des Innenrings 31 ist das Dichtelement 36 mit kreisförmigem Querschnitt, zum Beispiel ein Rundschnurdichtring aus Gummi, gehalten. Das Dichtelement 36 liegt mit seiner anderen Seite dicht an der Innenfläche des Dichtabschnitts 35 des Außenrings 33 an. Somit ist ein zwischen den Ringen 31 und 33 gebildeter Zwischenraum 37 ebenfalls dicht gegenüber der Außenseite der Dichtungsanordnung 30. Der Außenring 33 weist einen Anbringungsabschnitt 34 zur Anbringung an dem Gelenkendabschnitt 43 und den Dichtabschnitt 35 auf. Andere Formen sind selbstverständlich denkbar. Wichtig ist, dass die Oberflächen Sterilbarrierenabschnitte 25 und 26 über die Dichtungsanordnung 30 dicht und steril verbunden sind.

Eine Montage der Sterilbarriere S gestaltet sich besonders einfach und schnell. Dazu werden die einzelnen Sterilbarrierenabschnitte 24 bis 28 über die korrespondierenden Roboterabschnitte übergezogen. Dann werden die Innenringe 31 mit Schnellverschlüssen, die hier nicht gezeigt sind, befestigt. Es folgt ein Aufziehen der Dichtelemente 36 und der Außenringe 33, die auch Schnellverschlüsse aufweisen. Es wird mit dem Innenring 31 am Gelenk 12 (siehe Fig. 1) begonnen.

Die erfindungsgemäße Sterilbarriere S ist nicht auf die beschriebenen und in den Figuren dargestellten Ausführungen beschränkt. Es sind Modifikationen und Änderungen im Rahmen der beigefügten Ansprüche möglich.

Auch Knickgelenke können bei einer besonderen Ausgestaltung mit der erfindungsgemäßen Sterilbarriere S und der Dichtungsanordnung 30 ausgerüstet werden. Dazu müssen die Knickgelenke jeweils wenigstens Gelenkendabschnitte zur Montage der jeweiligen Endabschnitte der Sterilbarrierenabschnitte und Dichtungsanordnungen aufweisen.

## Patentansprüche

1. Sterilbarriere (S) für einen chirurgischen Roboter (1) mit zumindest einem Gelenk (12 - 15) mit zwei gegenüber liegenden, um eine gemeinsame Gelenkachse (16 - 19) relativ zueinander drehbaren Gelenkkörpern (40, 42) und einem Drehmomentsensor (29), aufweisend zumindest zwei Sterilbarrierenabschnitte (24 - 28) mit jeweils einem Endabschnitt (38, 39) zur dichten Anbringung an jeweils einem Gelenkendabschnitt (41, 43) der Gelenkkörper (40, 42), und eine Dichtungsanordnung (30) zur Bildung einer sterilen und dichten Drehverbindung der Endabschnitte (38, 39) der zumindest zwei Sterilbarrierenabschnitte (24 - 28),
**gekennzeichnet dadurch, dass**
die Dichtungsanordnung (30) einen Innenring (31) zur dichten Anbringung auf dem Gelenkendabschnitt (41) des ersten Gelenkkörpers (40) des Gelenks (12-15), einen Außenring (33) mit einem Anbringungsabschnitt (34) zur Anbringung auf dem Gelenkendabschnitt (43) des zweiten Gelenkkörpers (42) des Gelenks (12 - 15) und mit einem zur Überlappung des Innenrings (31) vorgesehenen Dichtabschnitt (35), und ein zur Anordnung zwischen dem Innenring (31) und dem Dichtabschnitt (35) des Außenrings (33) vorgesehenes Dichtelement (36) zur Abdichtung des Innenrings (31) gegenüber dem Außenring (33) aufweist, und die Endabschnitte (38, 39) der Sterilbarrierenabschnitte (25, 26) jeweils mit zumindest einem flexiblen Spannelement zur Fixierung und Zusammenwirkung mit einer korrespondierenden Ausnehmung an dem jeweiligen Gelenkendabschnitt (41, 43) ausgebildet sind.

2. Sterilbarriere (S) nach Anspruch 1, bei welcher der Innenring (31) und der Außenring (33) jeweils eine Verschlusseinrichtung zur Montage und Demontage aufweisen.

3. Sterilbarriere (S) nach Anspruch 1 oder 2, bei welcher der Innenring (31) und der Außenring (33) jeweils eine umlaufende Nase zur Zusammenwirkung mit einer jeweiligen Nut (44, 45) an dem korrespondierenden Gelenkendabschnitt (41, 43) aufweisen.

4. Sterilbarriere (S) nach einem der Ansprüche 1 bis 2, bei welcher die Endabschnitte (38, 39) der Sterilbarrierenabschnitte (25, 26) zur Zusammenwirkung mit korrespondierenden Anbringungsabschnitten (32, 34) des Innenrings (31) und Außenrings (33) zur Abdichtung und Befestigung an letzteren und den jeweiligen Gelenkendabschnitten (41, 43) vorgesehen sind.

5. Sterilbarriere (S) nach einem der Ansprüche 1 bis 4, bei welcher der Innenring (31) und Außenring (33) aus einem sterilisierbaren Werkstoff und/oder einer Kombination aus sterilisierbaren Werkstoffen ausgebildet sind.

6. Sterilbarriere (S) nach einem der Ansprüche 1 bis 5, bei welcher das Dichtelement (36) mit dem Innenring (31) oder mit dem Außenring (33) fest verbunden ist.

7. Sterilbarriere (S) nach einem der Ansprüche 1 bis 6, bei welcher das Dichtelement (36) ein steriler Zuschnitt oder ein sterilisierbarer Zuschnitt einer Endlosware ist.

8. Chirurgischer Roboter (1) mit zumindest einem Gelenk (12 - 15) mit einem Drehmomentsensor (29), welcher mit einer Sterilbarriere (S) nach einem der Ansprüche 1 bis 7 steril abgedeckt ist.

9. Chirurgischer Roboter (1) nach Anspruch 8, bei welchem zumindest ein Störgrößenbeobachter zur Erfassung eines Reibungswertes der Dichtungsanordnung (30) bei Drehbewegung und zur Weiterleitung des Reibungswertes an eine Regelungseinrichtung zur Kompensation der durch die Reibungskräfte hervorgerufenen Störungen der Messwerte des Drehmomentsensors (29) vorgesehen ist.

## Claims

1. Sterile barrier (S) for a surgical robot (1) comprising at least one joint (12 -15) with two opposite joint members (40, 42) which are rotatable relative to one another about a common joint axis (16 - 19) and a torque sensor (29) comprising at least two sterile barrier sections (24 - 28) each with an end section (38, 39) for sealing against an joint end section (41, 43) of the joint members (40, 42) and
a sealing arrangement (30) for forming a sterile and sealed rotary connection of the end sections (38, 39) of the at least two sterile barrier sections (24 - 28),
**characterised in that** the sealing arrangement (30) comprises an inner ring (31) for applying in a sealing manner onto the joint end section (41) of the first joint member (40) of the joint (12 -15),
an outer ring (33) with an attachment section (34) for attaching onto the joint end section (43) of the second joint member (42) of the joint (12 -15) and with a sealing section (35) provided for overlapping the inner ring (31), and a sealing element (36) for arranging between the inner ring (31) and the sealing section (35) of the outer ring (33) for sealing the inner ring (31) relative to the outer ring (33), and the end sections (38, 39) of the sterile barrier sections (25, 26) are each designed to have at least one flexible tensioning element for fixing and interacting with a corresponding recess on the respective joint end section (41, 43).

2. Sterile barrier (S) according to claim 1, wherein the inner ring (31) and the outer ring (33) each comprise a closure device for assembly and disassembly.

3. Sterile barrier (S) according to either claim 1 or claim 2, wherein the inner ring (31) and the outer ring (33) each have a peripheral nose for interacting with a respective groove (44, 45) on the corresponding joint end section (41, 43).

4. Sterile barrier (S) according to any one of claims 1 to 2, wherein the end sections (38, 39) of the sterile barrier sections (25, 26) are provided for interacting with corresponding attachment sections (32, 34) of the inner ring (31) and outer ring (33) for sealing and securing onto the latter and the respective articulation end sections (41, 43).

5. Sterile barrier (S) according to any one of claims 1 to 4, wherein the inner ring (31) and outer ring (33) are made from a sterilisable material and/or a combination of sterilisable materials.

6. Sterile barrier (S) according to any one of claims 1 to 5, wherein the sealing element (36) is securely connected to the inner ring (31) or the outer ring (33).

7. Sterile barrier (S) according to any one of claims 1 to 6, wherein the sealing element (36) is a sterile cut part or a sterilisable cut part of a continuous length of material.

8. Surgical robot (1) with at least one oint (12 - 15) with a torque sensor (29), which is covered in a sterile manner with a sterile barrier (S) according to any one of claims 1 to 7.

9. Surgical robot (1) according to claim 8, wherein at least one disturbance variable monitor is provided for detecting a friction value of the sealing arrangement (30) during the rotational movement and for transferring the friction value to a control device for compensating the disturbances of the measurement values of the torque sensor (29) caused by the friction forces.

## Revendications

1. Barrière stérile (S) pour un robot chirurgical (1), comportant au moins une articulation (12 - 15) avec deux corps d'articulation (40, 42) situés l'un en face de l'autre et rotatifs l'un par rapport à l'autre autour d'un axe d'articulation (16 - 19) commun, et un capteur de couple de rotation (29), présentant au moins deux tronçons de barrière stérile (24 - 28) avec un tronçon d'extrémité (38, 39) respectif pour être mis en place de manière étanche sur un tronçon d'extrémité d'articulation (41, 43) respectif des corps d'articulation (40, 42), et un dispositif d'étanchéité (30) pour former une liaison rotative stérile et étanche des tronçons d'extrémité (38, 39) desdits au moins deux tronçons de barrière stérile (24 - 28), **caractérisée en ce que** le dispositif d'étanchéité (30) présente une bague intérieure (31) pour la mise en place étanche sur le tronçon d'extrémité d'articulation (41) du premier corps d'articulation (40) de l'articulation (12 - 15), une bague extérieure (33) avec un tronçon de mise en place (34) pour la mise en place sur le tronçon d'extrémité d'articulation (43) du deuxième corps d'articulation (42) de l'articulation (12 - 15) et comportant un tronçon d'étanchéité (35) prévu pour le chevauchement de la bague intérieure (31), et un élément d'étanchéité (36) prévu pour être agencé entre la bague intérieure (31) et le tronçon d'étanchéité (35) de la bague extérieure (33) pour étancher la bague intérieure (31) par rapport à la bague extérieure (33), et **en ce que** les tronçons d'extrémité (38, 39) des tronçons de barrière stérile (25, 26) sont réalisés chacun avec au moins un élément de tension flexible pour la fixation et la coopération avec un évidement correspondant sur le tronçon d'extrémité d'articulation (41, 43) respectif.

2. Barrière stérile (S) selon la revendication 1, dans laquelle la bague intérieure (31) et la bague extérieure (33) présentent chacune un dispositif de fermeture pour le montage et le démontage.

3. Barrière stérile (S) selon la revendication 1 ou 2, dans laquelle la bague intérieure (31) et la bague extérieure (33) présentent chacune un taquet périphérique pour coopérer avec une gorge (44, 45) respective sur le tronçon d'extrémité d'articulation (41, 43) correspondant.

4. Barrière stérile (S) selon l'une des revendications 1 à 2, dans laquelle les tronçons d'extrémité (38, 39) des tronçons (25, 26) de barrière stérile sont prévus pour coopérer avec des tronçons de mise en place (32, 34) de la bague intérieure (31) et de la bague extérieure (33) pour l'étanchement et la fixation sur ces dernières et sur les tronçons d'extrémité (41, 43) respectifs.

5. Barrière stérile (S) selon l'une des revendications 1 à 4, dans laquelle la bague intérieure (31) et la bague extérieure (33) sont réalisées dans un matériau stérilisable et/ou dans une combinaison de matériaux stérilisables.

6. Barrière stérile (S) selon l'une des revendications 1 à 5, dans laquelle l'élément d'étanchéité (36) est relié de manière solidaire à la bague intérieure (31) ou à la bague extérieure (33).

7. Barrière stérile (S) selon l'une des revendications 1 à 6, dans laquelle l'élément d'étanchéité (36) est une découpe stérile ou une découpe stérilisable d'un produit sans fin.

8. Robot chirurgical (1) comportant au moins une articulation (12 - 15) avec un capteur de couple de rotation (29), qui est recouvert de façon stérile avec une barrière stérile (S) selon l'une des revendications 1 à 7.

9. Robot chirurgical (1) selon la revendication 8, dans lequel il est prévu au moins un observateur de grandeurs perturbatrices pour détecter une valeur de friction du dispositif d'étanchéité (30) lors d'un mouvement de rotation et pour transmettre la valeur de friction à un dispositif de réglage pour compenser les perturbations des valeurs de mesures du capteur de couple de rotation (29) provoquées par des forces de friction.
